# EUROPÄISCHE PATENTANMELDUNG

(11) **EP 2 058 397 A1**
(43) Veröffentlichungstag der Anmeldung: **13.05.2009**
(21) Anmeldenummer: 07075967.5
(22) Anmeldetag: 07.11.2007
(51) Int. Cl.: C12N 15/11

(54) **Multimeres Assemblat zur Immunstimulation**

(71) Anmelder: Mologen AG, 14195 Berlin (DE)
(72) Erfinder: Schroff, Matthias, 14195 Berlin (DE); Schmidt, Manuel, 14195 Berlin (DE); Wittig, Burghardt, 14129 Berlin (DE); Löhr, Janine, 12053 Berlin (DE); Kleuss, Christiane, 12203 Berlin (DE)
(74) Vertreter: Boeckh, Tobias

(57) **Zusammenfassung**

Die Erfindung betrifft ein multimeres, nicht-kodierendes Nukleinsäuremolekül zur Modulation der Aktivität des menschlichen oder tierischen Immunsystems sowie ein Herstellungsverfahren desselben und ein Vakzin, die das multimere, nicht-kodierende Nukleinsäuremolekül enthält, wobei multimere, nicht-kodierende Nukleinsäuremoleküle als nicht-kodierende Nukleinsäuremoleküle verstanden werden können, die aus mindestens zwei solcher Moleküle (Dimer) bestehen oder aber Assemblate aus mehreren nicht-kodierenden Nukleinsäuremolekülen.

## Beschreibung

Die Erfindung betrifft ein multimeres, nicht-kodierendes Nukleinsäuremolekül zur Modulation der Aktivität des menschlichen oder tierischen Immunsystems sowie ein Herstellungsverfahren desselben und ein Vakzin, die das multimere, nicht-kodierende Nukleinsäuremolekül enthält, wobei multimere, nicht-kodierende Nukleinsäuremoleküle als nicht-kodierende Nukleinsäuremoleküle verstanden werden können, die aus mindestens zwei solcher Moleküle (Dimer) bestehen oder aber Assemblate aus mehreren nicht-kodierenden Nukleinsäuremolekülen.

Während die adaptive Immunantwort nach Selektion der für das jeweilige Pathogen spezifischen Lymphozyten und deren klonaler Expansion und Differenzierung zu Effektorzellen erst verzögert (3-5 Tage) einsetzt und dann aber lang anhaltenden Schutz vor dem jeweiligen Pathogen durch Ausbildung eines "immunologischen Gedächtnisses" bietet, erkennen die Zellen des angeborenen Immunsystems Pathogene anhand konservierter Pathogen-assozüerter molekularer Muster (pathogenassociated molecular patterns = PAMPs) mit Keimzell-kodierten Rezeptoren und reagieren sofort. Zu den für verschiedene Zelltypen unterschiedlichen Reaktionen gehören die Sekretion von Cytokinen (z.B. IL-1, IL-6, TNF-α) und Chemokinen (z.B. IL-8/CXCL8, MIP-1α/β, MCP-1), Aktivierung von Effektormechanismen (Phagozytose, respiratorische Entladung, Freisetzung bakterizider Substanzen oder lytischer Granula), Expression von kostimulatorischen Molekülen (CD80, CD86) sowie verstärkter Expression von MHC-Molekülen. Dadurch werden zum Einen Effektorzellen rekrutiert und aktiviert, die das eingedrungene Pathogen eliminieren können, und zum Anderen erhalten die Zellen des adaptiven Immunsystems die für ihre Aktivierung notwendigen Signale.

Zur Erzeugung einer verbesserten Immunantwort wurden CpG-Oligonukleotide (CpG-ODN) als neue Klasse von immunmodulatorischen Molekülen eingesetzt. Solche nicht methylierte CG-Motive kommen in bakterieller DNA vor und stellen für das Immunsystem ein "danger signal" dar. Als "pathogen associated molecular pattern" (PAMP) bewirken sie vor allem die unspezifische Aktivierung des angeborenen Immunsystems (Krieg, Nat. Med 2003, 9: 831-835).

CpG-ODN induzieren über die Zytokine Interleukin-12, Interferon-gamma und Tumornekrosefaktor-alpha eine T_{H}1-betonte Immunantwort.

Immunstimulatorische Nukleinsäuresequenzen (ISS), die die benannten CpG-ODN tragen, sind nur einige Basen lang und wirken nicht über die Expression von auf ihnen kodierten Proteinen.

Bei den ISS handelt es sich um kovalent geschlossene Nukleinsäuremoleküle. Sie bestehen aus Oligonukleotiden, deren Basen partiell mit sich selbst paaren können und einer oder zwei Haarnadelschleifen (Loop), die 30 Basen umfassen und mehrere CG-Motive enthalten und im Weiteren als Trägermoleküle bezeichnet werden.

Die starke Stimulation der zellulären Immunantwort ermöglicht eine Einflussnahme auf Regelkreisläufe, die ohne Eingriff nicht zu einer für den Patienten befriedigenden Immunaktivität führen.

Die Modifikation von CpG-ODN mit Phosphorothioat-Rückgrat, wie sie zur Stabilisierung von "CpG-DNA" eingesetzt wird, weist einige gravierende Nachteile auf. Hierzu gehören insbesondere die beobachtete Toxizität [Heikenwalder 2004, Levin 1999] sowie unspezifische Bindung an Proteine [Brown 1994].

Aus diesem Grund wurde eine neue Klasse kovalent geschlossener immunstimulatorischer DNA entwickelt (EP 1196178). Diese DNA-Moleküle bestehen aus zwei chemisch synthetisierten DNA-Molekülen, die einen selbstkomplementären Bereich am 5'- und am 3'-Ende mit palindromischen, überlappenden Enden aufweisen, so dass durch Ligation der beiden DNA-Moleküle ein kovalent geschlossenes Molekül entsteht. Diese DNA-Moleküle mit CG-Motiven im nicht-komplementären Bereich zeigen ähnliche Aktivität wie CpG-ODN (verstärkte Expression der Oberflächenmoleküle CD80, CD40, MHC auf B-Zellen und Sekretion von IL-6, IFN-γ, IFN-α IL-12, TNFα durch PBMC), weisen aber im Vergleich zu CpG-ODN mit Phosphorothioat-Rückgrat Unterschiede im Induktionsmuster der Zytokine und eine deutlich geringere Toxizität in Mäusen auf. Diese immunstimulatorische DNA des Standes der Technik weist bezüglich der Modulation der Aktivität des menschlichen oder tierischen Immunsystems aber einige Nachteile auf. Es ist nicht immer möglich, die Aktivität des menschlichen oder tierischen Immunsystems in der gewünschten Stärke zu modulieren, insbesondere zu aktivieren.

Ausgehend von diesem Stand der Technik ist es die Aufgabe der vorliegenden Erfindung geeignete immunstimulatorische DNA-Moleküle, die zur Auslösung einer verbesserten Immunantwort fähig sind, sowie ein Verfahren zu ihrer Herstellung sowie Impfstoffe bereitzustellen, die diese immunstimulatorischen DNA-Moleküle enthalten.

Immunstimulation heißt im Zusammenhang dieser Erfindung, dass die Mittler- und Effektorzellen des Immunsystems, also vor allem die zur Zeit bekannten Thymozyten mit Helferfunktion und die cytotoxischen Thymozyten, B-Zellen und sog. NK (natural killer)-Zellen, Makrophagen und Monozyten sowie dendritische Zellen und ihre Vorläufer, sowie bisher in ihrer Funktion nicht aufgeklärte Zellpopulationen mit Funktionen innerhalb des Immunsystems, durch die Verwendung von Nukleinsäuremolekülen zur Proliferation, Migration, Differenzierung oder Aktivität angeregt werden. Immunmodulation heißt, dass neben einer generellen Stimulation im oben definierten Sinne auch die Art oder der Charakter einer Immunreaktion beeinflusst wird, sei es, dass eine im Entstehen oder der Reifung begriffene Immunreaktion davon betroffen ist, sei es, dass eine schon etablierte Reaktion in ihrem Charakter verändert wird.

Die vorliegende Erfindung löst die Aufgabe dadurch, dass sie ein multimeres, nicht-kodierendes Nukleinsäuremolekül zur Verfügung stellt. Es war völlig überraschend, dass Dimere, Trimere, Pentamere oder Multimere von kovalent geschlossener immunstimulatorischer DNA einen überraschend verbesserten Effekt gegenüber den Molekülen des Standes der Technik haben. Das erfindungsgemäße multimere Molekül ist herstellbar durch ein Verfahren, welches die folgenden Schritte umfasst:
- Bereitstellung einer Polyacrylamidgelelektrophorese-gereinigten, 5-phosphorylierten Oligodesoxyribonukleinsäuresequenz in Wasser,
- Lyophylisierung bis ein trockener Rückstand erhalten wird und anschließend Resuspension in einer Pufferlösung,
- Zugabe einer T4-DNAligase, wodurch ein Reaktionsgemisch gebildet wird und
- Inkubation des Reaktionsgemisches bei 37°C für mindestens 30 Minuten.

Statt der Reinigung der Nukleinsäuresequenz durch eine Polyacrylamidgelelektrophorese (PAGE) kann auch eine Nukleinsäuresequenz mit dem Reinigungsgrad einer PAGE verwendet werden. Das heißt, die Nukleinsäuresequenz ist über ein anderes Verfahren so gereinigt worden, wie das der Fall gewesen wäre, wenn sie über eine PAGE gereinigt worden wäre. Ein im Sinne der Erfindung äquivalenter Reinigungsgrad zum PAGE-Reinigungsgrad wird beispielsweise durch die kombinierte Reinigung der Nukleinsäuresequenzen zunächst mit einer HPLC gefolgt von einer FPLC erreicht (eine Reinigung nur mit einer HPLC führt nicht zu den erfindungsgemäßen multimeren Komplexen, da diese nicht zu einem Reinigungsgrad führt, der äquivalent zu dem Reinigungsgrad einer PAGE ist). Ein äquivalenter Reinigungsgrad führt wie der PAGE-Reinigungsgrad zu mindestens einer Dimerbildung der Nukleinsäuresequenz/Oligodesoxyribonukleinsäuresequenz. Überraschenderweise führt die Abfolge der einzelnen Verfahrensschritte zu einem multimeren Molekül, welches die Aktivität des menschlichen oder tierischen Immunsystems besser moduliert, insbesondere aktiviert, als Moleküle des Standes der Technik. Die Moleküle des Standes der Technik sind die bekannten immunstimulatorischen Nukleinsäuresequenzen, die in der Regel im Stand der Technik nur einige Basen lang sind und nicht über die Expression der von ihnen kodierten Proteine wirken. Die meisten bekannten, immunmodifizierenden kurzen Oligodesoxyribonukleinsäuresequenzen enthalten ein unmethyliertes Cytosin-Guanosin-Motiv. Die physiologische Wirkung dieser Nukleinsäuresequenzen wird als Immunmodulation bzw. Modulation der Aktivität des Immunsystems im Sinne der Erfindung verstanden. In der EP 1 196 178 werden beispielsweise verschiedene Moleküle offenbart, die aus einem Stamm und mindestens einer Schleife bestehen, wie sie beispielsweise in den Figuren 1, 2 und 3 offenbart sind. Im Sinne der Erfindung handelt es sich bei diesen Molekülen um eine Monomerstruktur. Hierbei ist zu berücksichtigen, dass der Begriff des Oligomers in der Wissenschaft mehrfach mit unterschiedlichen Bedeutungen verwendet wird. Ein Oligomer kann beispielsweise eine längere Nukleinsäuresequenz sein, aber auch eine Struktur, die aus mehreren gleichen oder ähnlichen Molekülen besteht und zu einem größeren Assemblat zusammengefügt wurde. Ein Multimer im Sinne der Erfindung wären beispielsweise mehrere Stamm-Schleifen-Strukturen gemäß der EP 1 196 178, die mit mehreren gleichen oder ähnlichen Stamm-Schleifen-Strukturen zu einer höheren Struktur (einem Multimer) zusammengefügt wurden. Es ist überraschend, dass derartige oligomere Strukturen, die verbesserte und nicht naheliegende Eigenschaften gegenüber den monomeren Strukturen haben, durch vergleichsweise einfache Verfahrensschritte gewonnen werden können. Die Gewinnung der Assemblate lässt sich beispielsweise durch Zentrifugation, Gelelektrophorese oder Chromatographiesäulen feststellen, mithilfe derer hochkomplexe Strukturen, wie beispielsweise Dimere, Pentamere oder andere, nachgewiesen und gewonnen werden können, die gegenüber den monomeren Strukturen überraschend bessere Eigenschaften bei der Modulation des Immunsystems zeigen. Wenn bei dem erfindungsgemäßen Verfahren auf die Reinigung mit der PAGE oder auf eine äquivalente Art Reinigung der Oligodesoxyribonukleinsäuersequenzen verzichtet wird, werden multimere Strukturen gewonnen, die von den anmeldungsgemäßen verschieden sind. Dies zeigt sich darin, dass sie im Labororganismus oder beim Menschen zu einer anderen Form der Immunstimulation führen.

In einer bevorzugten Ausführungsform der Erfindung ist das Multimer bzw. das Assemblat gemäß der Erfindung dadurch gekennzeichnet, dass die im Verfahren eingesetzte Oligodesoxyribonukleinsäuresequenz die folgenden Sequenzen umfasst:
a) CTGCAGCTGTAGCAGCTTCGGGGGGTATCGTTCTTCGTGTCGTTCT-TAGCTGCTACAG und/oder
b) AGGGGTTACCACCTTCTATAGAAAACGGTTCTTCGGGGCGTTCTT-CATCGGTAACCCCT und/oder
c) eine Oligodesoxyribonukleinsäuresequenz der Basenfolge CCTAGGGGT-TACCACCTTCATTGGAAAACGTTCTTCGGGGCGTTCTTAGGTGGTAACC und/oder
d) CTAGGGGTTACCACCTTCATTGGAAAACGTTCTTCGGGGCGTTCT-TAGGTGGTAACCC und/oder
e) CTGAGGGTTACCACCTTCATTGGAAAACGTTCTTCGGGGCGTTCT-TAGGTGGTAACCC und/oder TCAGGGGTTACCACCTTCATTGGAA-AACGTTCTTCGGGGCGTTCTTAGGTGGTAACCC umfasst,
f) GCCGGGGTTACCACCTTCATTGGAAAACGTTCTTCGGGGCGTTCT-TAGGTGGTAACCC und CGGCGGGTTACCACCTTCATTGGAA-AACGTTCTTCGGGGCGTTCTTAGGTGGTAACCC und/oder
g) ATTAGGGTTACCACCTTCATTGGAAAACGTTCTTCGGGGCGTTCT-TAGGTGGTAACCC und TAATGGGTTACCACCTTCATTGGAA-AACGTTCTTCGGGGCGTTCTTAGGTGGTAACCC und/oder
h) CCTGAGGGTTACCACCTTCATTGGAAAACGTTCTTCGGGGCGTTCT-TAGGTGGTAACC und CTCAGGGGTTACCACCTTCATTGGAA-AACGTTCTTCGGGGCGTTCTTAGGTGGTAACC,
i) und wobei die Oligodesoxyribonukleinsäuresequenz eine Länge von 20 bis 400 Nukleotide aufweist.

Besonders bevorzugt ist es, wenn die Sequenzen gemäß e) beide verwendet werden.

Die Auswahl der bevorzugten Sequenzen führt zu Molekülen, die überraschend gut zur Stimulierung des Immunsystems eingesetzt werden können. Besonders bevorzugt ist es, wenn die Basenfolge gemäß Merkmal c) in der Sequenz CCTAGGGGT-TACCACCTTCATTGGAAAACGTTCTTCGGGGCGTTCTTAGGTGGTAACCCC-TAGGGGTTACCACCTTCATTGGAAAACGTTCTTCGGGGCGTTCTTAGGTGG-TAACC bzw. nach e) CTGAGGGTTACCACCTTCATTGGAA-AACGTTCTTCGGGGCGTTCTTAGGTGGTAACCCTCAGGGGTTACCACCTT-CATTGGAAAACGTTCTTCGGGGCGTTCTTAGGTGGTAACCC enthalten ist. Überraschenderweise führt das Vorhandensein dieser Sequenzen zu einer besonders guten Immunstimulierung in einem Zielorganismus.

In einer weiteren bevorzugten Ausführungsform der Erfindung ist vorgesehen, dass das Molekül eine teilweise einsträngige, kovalent geschlossene Kette von Desoxyribonukleotidresten umfasst. Innerhalb der assemblierten oligomeren bzw. polymeren Struktur des Moleküls ist die teilweise einsträngig kovalent geschlossene Kette von Desoxyribonukleotidresten dafür verantwortlich, dass das Molekül in dem Zielorganismus, in den es eingebracht wird, über einen längeren Zeitraum effektiv wirkt.

In einer weiteren bevorzugten Ausführungsform der Erfindung ist vorgesehen, dass das Molekül die Basenfolge N¹N²CGN³N⁴ umfasst, wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist.

In einer ganz besonders bevorzugten Ausführungsform ist vorgesehen, dass die Basenfolge N¹N²CGN³N⁴ in dem einsträngigen Bereich der geschlossenen Kette von Desoxyribonukleotidresten positioniert ist. Insbesondere diese bevorzugten Moleküle zeigen eine sehr effektive Wirkung bei der Stimulierung des Immunsystems.

Selbstverständlich kann es vorgesehen sein, dass das erfindungsgemäße Molekül mit einem oder mehreren Substituenten durch kovalente Bindung verknüpft vorliegt. Bei diesen Substituenten kann es sich beispielsweise um Peptide, Proteine, Sacharide, antigene Strukturen, DNA und/oder RNA handeln, besonders bevorzugt sind: Aminosäuren, Monosaccharide, Fettsäuren, Desoxy- und Ribonukleotide in ihrer monomeren oder polymeren Form, einschließlich Lipide.

Die Erfindung betrifft auch ein Kombinationsmittel, welches mindestens ein erfindungsgemäßes Molekül und ein Chemotherapeutikum umfasst. Es war unerwartet, dass die überraschend hohe Stimulation des Immunsystems durch das erfindungsgemäße Molekül noch einmal verbessert werden kann, wenn das erfindungsgemäße Mittel mit bekannten Chemotherapeutika kombiniert wird und das Kombinationsmittel gegen Tumoren eingesetzt wird. Das Kombinationsmittel im Sinne der Erfindung kann auch als Kit vorliegen, in welchem das erfindungsgemäße Molekül und das Chemotherapeutikum gemäß des Standes der Technik getrennt vorliegen. So können in bevorzugten Ausführungsformen die mindestens zwei Bestandteile des Kits zeitgleich oder zeitversetzt appliziert werden. Beispielsweise kann die Gabe des erfindungsgemäßen Kombinationsmittels das Immunsystem so aktivieren, dass eine nachfolgende Applikation eines Chemotherapeutikums seine Wirkung besonders effektiv entfalten kann. Selbstverständlich ist es aber auch möglich, dass zunächst das Chemotherapeutikum appliziert und anschließend zeitlich versetzt davon das erfindungsgemäße Molekül in den humanen oder tierischen Organismus gegeben wird. Bei bestimmten Tumoren ist die zeitgleiche Gabe von erfindungsgemäßen Molekül und Chemotherapeutikum bevorzugt.

In einer bevorzugten Ausführungsform der Erfindung ist das Chemotherapeutikum ausgewählt aus der Gruppe umfassend Antikörper, Alkylantien, Platinanaloga, Interkalentien, Antibiotika, Mitosehemmer, Taxane, Topoisomerasehemmer, Antimetabolite und/oder L-Asparaginase, Hydroxycarbamid, Mitotane und/oder Amanitine.

In einer bevorzugten Ausführungsform der Erfindung sind die Alkylantien ausgewählt aus der Gruppe umfassend
- Stickstoff-Lost-Derivate, insbesondere
- Cyclophosphamid,
- Ifosfamid,
- Trofosfamid,
- Melphalan und/oder
- Chlorambucil
- Akylsulfonate, insbesondere
- Busulfan und/oder
- Treosulfan
- Nitrosoharnstoffe, insbesondere
- Carmustin,
- Lomustin,
- Nimustin,
- Estramustin und/oder
- Streptozotocin
- Procarbazin und Dacarbazin,
- Temozolomid und/oder
- Thiotepa.

Die Alkylantien wirken besonders gut auf Tumoren, wodurch sie deren Wachstum inhibieren.

In einer bevorzugten Ausführungsform der Erfindung sind die Platinanaloga ausgewählt aus der Gruppe umfassend:
- Cisplatin,
- Carboplatin und/oder
- Oxaliplatin.

In einer weiteren bevorzugten Ausführungsform der Erfindung ist vorgesehen, dass die Interkalentien ausgewählt sind aus der Gruppe umfassend:
- Anthracycline, insbesondere
- Doxorubicin (Adriamycin),
- Daunorubicin,
- Epirubicin und/oder
- Idarubicin,
- Mitoxantron,
- Amsacrin und/oder
- Doxifluridin

In einer weiteren bevorzugten Ausführungsform der Erfindung ist vorgesehen, dass die Antibiotika ausgewählt sind aus der Gruppe umfassend:
- Bleomycin,
- Actinomycin D (Dactinomycin) und/oder
- Mitomycin

Weiterhin kann es in einer anderen bevorzugten Ausführungsform der Erfindung vorteilhaft sein, dass die Mitosehemmer ausgewählt sind aus der Gruppe umfassend:
- Alkaloide der Vinca rosea, insbesondere
- Vinorelbin,
- Vincristin (Oncovin),
- Vinblastin und/oder
- Vindesin

In einer weiter besonders bevorzugten Ausführungsform der Erfindung sind die Taxane ausgewählt aus der Gruppe umfassend:
- Paclitaxel und/oder
- Docetaxel.

Weiterhin kann es bevorzugt sein, dass die Topoisomerasehemmer ausgewählt sind aus der Gruppe umfassend:
- Topoisomerase-I-Inhibitoren, insbesondere
- Camptothecin,
- Topotecan und/oder
- Irinotecan und/oder
- Topoisomerase-II-Inhibitoren, insbesondere
- Etoposid
- Teniposid.

Weiterhin ist es bevorzugt, dass in einer besonderen Ausführungsform der Erfindung die Antimetabolite ausgewählt sind aus der Gruppe umfassend:
- Folsäureantagonist, insbesondere
- Methotrexat,
- Pyrimidinanaloga, insbesondere
- 5-Fluorouracil,
- Capecitabin,
- Cytosinarabinosid (Cytarabin) und/oder
- Gemcitabin,
- Purinanaloga, insbesondere
- 6-Thioguanin,
- Pentostatin,
- Azathioprin,
- 6-Mercaptopurin,
- Fludarabin und/oder
- Cladribin.

Die Erfindung betrifft auch einen Kit, der das erfindungsgemäße Molekül und das Chemotherapeutikum umfasst, gegebenenfalls mit einer Information zum Kombinieren der Inhalte des Kits. Die Erfindung betrifft aber auch - wie bereits ausgeführt - ein pharmazeutisches Mittel, welches das erfindungsgemäße Molekül oder das Kombinationsmittel gegebenenfalls mit einem pharmazeutisch verträglichen Träger umfasst.

Die Erfindung betrifft weiterhin die Verwendung des Moleküls, des Kombinationsmittel oder des pharmazeutischen Mittels zur Herstellung eines Mittels zur Modulation eines menschlichen oder tierischen Immunsystems oder zur Modulation der Aktivität dieses Immunsystems. Unter Modulation des menschlichen oder tierischen Immunsystems wird jeder Einfluss auf das Immunsystem verstanden, der dazu führt, dass das Immunsystem auf Tumoren bzw. auf Krebs insbesondere inhibierend wirkt. Die Modulation der Aktivität des Immunsystems kann hierzu synonym verstanden werden oder sie beschreibt die dem Fachmann bekannten Aktivitäten des Immunsystems, die gegen Tumoren gerichtet sind und die durch die erfindungsgemäßen Mittel in Ihre Aktivität überraschend gesteigert werden. Die Modulation ist daher insbesondere eine Stimulierung oder Steigerung von Wirkungen des Immunsystems bzw. des Immunsystems selbst. So können die erfindungsgemäßen Mittel in einer bevorzugten Ausführungsform dazu verwendet werden, die T-Zell vermittelte Immunantwort aber auch die T-Zell unabhängige Immunantwort zu stimulieren. Dieser Vorgang kann in einer bevorzugten Ausführungsform der Erfindung eine Proliferation von B-Zellen umfassen oder eine B-Zell-Aktivierung.

In einer ganz besonders bevorzugten Ausführungsform kommt es bei der Modulation der Aktivität des Immunsystems zu einer Stimulierung dergestalt, dass Zytokine ausgeschüttet bzw. verstärkt ausgeschüttet werden. Es kann besonders bevorzugt sein, wenn das erfindungsgemäße Molekül bzw. das Kombinationsmittel gemäß der Erfindung als Adjuvanz in der therapeutischen oder prophylaktischen Vakzinierung eingesetzt werden. Besonders wirksam können die erfindungsgemäßen Mittel zur Behandlung einer Zellwachstumsstörung eingesetzt werden, wobei in einer bevorzugten Ausführungsform die Zellwachstumsstörung eine Tumorerkrankung ist. Bevorzugt handelt es sich bei der Tumorerkrankung um eine Krankheit, die ausgewählt ist aus der Gruppe umfassend Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nichtkleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assozüerte Lymphome, AIDS-assozüerte Lymphome des zentralen Nervensystems, AIDS-assozüerter Morbus Hodgkin und AIDS-assozüerter anogenitale Tumoren, Transplantations-bezogene Malignitäten, metastasierte Tumoren umfassend Gehirnmetastasen, Lungen-metastasen, Lebermetastasen, Knochenmetastasen, pleurale und perikardiale Metastasen und maligne Aszites.

Im folgenden soll die Erfindung an Hand von Beispielen näher erläutert werden, ohne auf diese Beispiele beschränkt zu sein.

Die Ausbildung der hantelförmigen Monomere ist dem Fachmann aus dem Stand der Technik bekannt. [EP 1 196 178] Die hierfür eingesetzte 5-phosphorylierten Oligodesoxyribonukleinsäuresequenz (CCTAGGGGTTACCACCTTCATTGGAA-AACGTTCTTCGGGGCGTTCTTAGGTGGTAACC) wurde über eine PAGE gereinigt und in Wasser bereitgestellt. Anschließend erfolgte eine Lyophilisierung, nach deren Abschluss der trockene Rückstand in einer Pufferlösung resuspendiert wurde. Gemäß der o. g. Vorschrift wurde eine T4-DNAligase zugegeben und es erfolgte die Ligation bei 37°C für 30 Minuten. Zum Vergleich wurde das Verfahren noch einmal ohne die Lyophilisierung und ohne die Reinigung der Oligodesoxyribonukleinsäuresequenz über eine PAGE durchgeführt. Der Einsatz der PAGE gereinigten Nukleinsäuresequenzen führt zur Ausbildung von Dimeren und Tetrameren, die im Sinne der Erfindung Multimere sind. Anschließend wurde die Wirkung der Monomere und der Multimere in der Maus getestet. Den Tieren wurde das entsprechende Material injiziert und es wurde nach 6 Wochen Blut abgenommen, dessen Serum zentrifugiert und gelagert wurde. Die Proben wurden gemeinsam auf IL-12 mit ELISA getestet. Weiterhin wurde die verbesserte Reifung von dendritischen Zellen unter Einwirkung der gewonnenen Konstrukte getestet. Hierbei zeigte sich, dass die erfindungsgemäßen Moleküle gegenüber den bekannten Vorteile bei der Modulation der Immunreaktion aufweisen.

## Patentansprüche

1. Oligomeres Molekül zur Modulation der Aktivität des menschlichen oder tierischen Immunsystems herstellbar durch ein Verfahren, welches folgende Schritte umfasst:
- Bereitstellung einer Polyacrylamidgelelektrophorese-gereinigten, 5-phosphorylierten Oligodesoxyribonukleinsäuresequenz in Wasser,
- Lyophylisierung bis ein trockener Rückstand erhalten wird und anschließend Resuspension in einer Pufferlösung,
- Zugabe einer T4-DNAligase, wodurch ein Reaktionsgemisch gebildet wird und
- Inkubation des Reaktionsgemisches bei 37°C für mindestens 30 Minuten.

2. Molekül nach Anspruch 1, **dadurch gekennzeichnet, dass** die Oligodesoxyribonukleotidsequenz die folgenden Sequenzen umfasst:
a) CTGCAGCTGTAGCAGCTTCGGGGGG-TATCGTTCTTCGTGTCGTTCTTAGCTGCTACAG und/oder
b) AGGGGTTACCACCTTCTATAGAAAACGGTTCTTCGGGGCGTTCTT-CATCGGTAACCCCT und/oder
c) eine Oligodesoxyribonukleinsäuresequenz der Basenfolge CCTAGGGGTTACCACCTTCATTGGAA-AACGTTCTTCGGGGCGTTCTTAGGTGGTAACC und/oder
d) CTAGGGGTTACCACCTTCATTGGAAAACGTTCTTCGGGGCGTTCT-TAGGTGGTAACCC und/oder
e) CTGAGGGTTACCACCTTCATTGGAAAACGTTCTTCGGGGCGTTCT-TAGGTGGTAACCC und/oder TCAGGGGTTACCACCTTCATTGGAA-AACGTTCTTCGGGGCGTTCTTAGGTGGTAACCC umfasst,
f) GCCGGGGTTACCACCTTCATTGGAAAACGTTCTTCGGGGCGTTCT-TAGGTGGTAACCC und CGGCGGGTTACCACCTTCATTGGAA-AACGTTCTTCGGGGCGTTCTTAGGTGGTAACCC und/oder
g) ATTAGGGTTACCACCTTCATTGGAAAACGTTCTTCGGGGCGTTCT-TAGGTGGTAACCC und TAATGGGTTACCACCTTCATTGGAA-AACGTTCTTCGGGGCGTTCTTAGGTGGTAACCC und/oder
h) CCTGAGGGTTACCACCTTCATTGGAA-AACGTTCTTCGGGGCGTTCTTAGGTGGTAACC und CTCAGGGGT-TACCACCTTCATTGGAAAACGTTCTTCGGGGCGTTCTTAGGTGG-TAACC,
i) und wobei die Oligodesoxyribonukleinsäuresequenz eine Länge von 20 bis 400 Nukleotide aufweist.

3. Molekül nach dem vorhergehenden Anspruch, **dadurch gekennzeichnet, dass** die Basenfolge gemäß Merkmal c) in der Sequenz CCTAGGGGTTAC-CACCTTCATTGGAAAACGTTCTTCGGGGCGTTCTTAGGTGGTAACCCC-TAGGGGTTACCACCTTCATTGGAAAACGTTCTTCGGGGCGTTCT-TAGGTGGTAACC bzw. nach e) CTGAGGGTTACCACCTTCATTGGAA-AACGTTCTTCGGGGCGTTCTTAGGTGGTAACCCTCAGGGGTTAC-CACCTTCATTGGAAAACGTTCTTCGGGGCGTTCTTAGGTGGTAACCC enthalten ist.

4. Molekül nach einem der vorhergehenden Ansprüche, **dadurch gekennzeichnet, dass** das Molekül eine teilweise einsträngige, kovalent geschlossene Kette von Desoxyribonukleotidresten umfasst.

5. Molekül nach einem der vorgehenden Ansprüche, **dadurch gekennzeichnet, dass** das Molekül die Basenfolge N¹N²CGN³N⁴ umfasst, wobei N¹N² ein Element aus der Gruppe GT, GG, GA, AT oder AA, N³N⁴ ein Element aus der Gruppe CT oder TT, sowie C Desoxycytosin, G Desoxyguanosin, A Desoxyadenosin und T Desoxythymidin ist.

6. Molekül nach einem der vorgehenden Ansprüche, **dadurch gekennzeichnet, dass** die Basenfolge N¹N²CGN³N⁴ in dem einsträngigen Bereich der geschlossenen Kette von Desoxyribonukleotidresten positioniert ist.

7. Kombinationsmittel nach einem der vorhergehenden Ansprüchen, **dadurch gekennzeichnet, dass** das Chemotherapeutikum ausgewählt ist aus der Gruppe umfassend Antikörper, Alkylantien, Platinanaloga, Interkalentien, Antibiotika, Mitosehemmer, Taxane, Topoisomerasehemmer, Antimetabolite und/oder L-Asparaginase, Hydroxycarbamid, Mitotane, und/oder Amanitine.

8. Kombinationsmittel nach einem der vorhergehenden Ansprüchen, **dadurch gekennzeichnet, dass** die Alkylantien ausgewählt sind aus der Gruppe umfassend:
- Stickstoff-Lost-Derivate, insbesondere
- Cyclophosphamid,
- Ifosfamid,
- Trofosfamid,
- Melphalan und/oder
- Chlorambucil
- Akylsulfonate, insbesondere
- Busulfan und/oder
- Treosulfan
- Nitrosoharnstoffe, insbesondere
- Carmustin,
- Lomustin,
- Nimustin,
- Estramustin und/oder
- Streptozotocin
- Procarbazin und Dacarbazin,
- Temozolomid und/oder
- Thiotepa.

9. Kombinationsmittel nach einem der vorhergehenden Ansprüche,
**dadurch gekennzeichnet, dass**
Platinanaloga ausgewählt sind aus der Gruppe umfassend:
-Cisplatin,
- Carboplatin und/oder
- Oxaliplatin.

10. Kombinationsmittel nach einem der hervorgehenden Ansprüche,
**dadurch gekennzeichnet, dass**
die Interkalentien ausgewählt sind aus der Gruppe umfassend:
- Anthracycline, insbesondere
- Doxorubicin (Adriamycin),
- Daunorubicin,
- Epirubicin und/oder
- Idarubicin,
- Mitoxantron,
- Amsacrin und/oder
- Doxifluridin.

11. Kombinationsmittel nach einem der hervorgehenden Ansprüche,
**dadurch gekennzeichnet, dass**
die Antibiotika ausgewählt sind aus der Gruppe umfassend:
- Bleomycin,
- Actinomycin D (Dactinomycin) und/oder
- Mitomycin.

12. Kombinationsmittel nach einem der hervorgehenden Ansprüche,
**dadurch gekennzeichnet, dass**
die Mitosehemmer ausgewählt sind aus der Gruppe umfassend:
- Alkaloide der Vinca rosea, insbesondere
- Vinorelbin,
- Vincristin (Oncovin),
- Vinblastin und/oder
- Vindesin.

13. Kombinationsmittel nach einem der hervorgehenden Ansprüche,
**dadurch gekennzeichnet, dass**
die Taxane ausgewählt sind aus der Gruppe umfassend:
- Paclitaxel und/oder
- Docetaxel.

14. Kombinationsmittel nach einem der vorhergehenden Ansprüche,
**dadurch gekennzeichnet, dass**
die Topoisomerasehemmer ausgewählt sind aus der Gruppe umfassend:
- Topoisomerase-I-Inhibitoren, insbesondere
- Camptothecin,
- Topotecan und/oder
- Irinotecan und/oder
- Topoisomerase-II-Inhibitoren, insbesondere
- Etoposid
- Teniposid.

15. Kombinationsmittel nach einem der hervorgehenden Ansprüche,
**dadurch gekennzeichnet, dass**
die Antimetabolite ausgewählt sind aus der Gruppe umfassend:
- Folsäureantagonist, insbesondere
- Methotrexat,
- Pyrimidianaloga, insbesondere
- 5-Fluorouracil,
- Capecitabin,
- Cytosinarabinosid (Cytarabin) und/oder
- Gemcitabin,
- Purinanaloga, insbesondere
- 6-Thioguanin,
- Pentostatin,
- Azathioprin,
- 6-Mercaptopurin,
- Fludarabin und/oder
- Cladribin.

16. Kit umfassend das Molekül nach einem der Ansprüche 1 bis 6 und/oder das Kombinationsmittel nach einem der Ansprüche 7 bis 15 und ggf. eine Information zum Kombinieren der Inhalte des Kit.

17. Molekül nach einem der Ansprüche 1 bis 6, Kombinationsmittel nach einem der Ansprüche 7 bis 15 zur Verwendung als Arzneimittel.

18. Pharmazeutisches Mittel umfassend ein Molekül nach einem der Ansprüche 1 bis 6 und/oder ein Kombinationsmittel nach einem der Ansprüche 7 bis 15 gegebenenfalls mit einem pharmazeutisch verträglichen Träger.

19. Pharmazeutisches Mittel nach dem vorhergehenden Anspruch, **dadurch gekennzeichnet, dass** der Träger ausgewählt ist aus der Gruppe umfassend Antikörper, Alkylantien, Platinanaloga, Interkalentien, Antibiotika, Mitosehemmer, Taxane, Topoisomerasehemmer, Antimetabolite und/oder L-Asparaginase, Hydroxycarbamid, Mitotane, und/oder Amanitine.

20. Verwendung eines Moleküls gemäß der Ansprüche 1 bis 6, eines Kombinationsmittel gemäß einem der Ansprüche 7 bis 15, eines Pharmazeutisches Mittel gemäß des Anspruchs 18 oder 19 zur Herstellung eines Mittels zur Modulation eines menschlichen oder tierischen Immunsystems oder zur Modulation der Aktivität des Immunsystems.

21. Verwendung nach dem vorhergehenden Anspruch, **dadurch gekennzeichnet, dass** die Modulation eine Stimulierung oder eine Steigerung der Aktivität des Immunsystems ist.

22. Verwendung nach dem vorhergehenden Anspruch, **dadurch gekennzeichnet, dass** die Stimulierung eine T-Helferzelle vermittelte oder-unabhängige Immunantwort umfasst.

23. Verwendung nach dem vorhergehenden Anspruch, **dadurch gekennzeichnet, dass** die Immunantwort eine Proliferation von B-Zellen umfasst und/oder eine B-Zell-Aktivierung.

24. Verwendung nach einem der vorhergehenden Ansprüche, **dadurch gekennzeichnet, dass** die Stimulierung des Immunsystems eine Sekretion von Zytokinen umfasst.

25. Verwendung nach einem der vorhergehenden Ansprüche, **dadurch gekennzeichnet, dass** das Molekül gemäß einem der Ansprüche 1 bis 6 und/oder das Kombinationsmittel gemäß einem der Ansprüche 7 bis 15 als Adjuvanz in der therapeutischen oder prophylaktischen Vakzinierung eingesetzt wird.

26. Verwendung eines Moleküls gemäß einem der Ansprüche 1 bis 6, eines Kombinationsmittels gemäß einem der Ansprüche 7 bis 15 und/oder eines pharmazeutischen Mittels gemäß des Anspruchs 18 oder 19 zur Herstellung eines Mittels zur Behandlung einer Zellwachstumsstörung.

27. Verwendung nach dem vorhergehenden Anspruch, **dadurch gekennzeichnet, dass** die Zellwachstumsstörung eine Tumorerkrankung ist.
